(19) Europäisches Patentamt
European Patent Office
Office européen des brevets

(11) Veröffentlichungsnummer: **0 114 359**
**B1**

# (12) EUROPÄISCHE PATENTSCHRIFT

(45) Veröffentlichungstag der Patentschrift: **31.07.85**

(21) Anmeldenummer: **83112901.0**

(22) Anmeldetag: **21.12.83**

(51) Int. Cl.⁴: **C 07 C 17/32**, C 07 C 21/24, C 07 C 25/13, C 07 D 213/26, C 07 D 307/38, C 07 C 41/30, C 07 C 43/225, C 07 C 67/343, C 07 C 69/76

## (54) Verfahren zur Herstellung von perfluoralkylsubstituierten carbocyclischen oder heterocyclischen Verbindungen.

(30) Priorität: **23.12.82 DE 3247728**

(43) Veröffentlichungstag der Anmeldung: **01.08.84 Patentblatt 84/31**

(45) Bekanntmachung des Hinweises auf die Patenterteilung: **31.07.85 Patentblatt 85/31**

(84) Benannte Vertragsstaaten: **BE CH DE FR GB IT LI NL**

(56) Entgegenhaltungen:
**DE - B - 1 049 862**
**US - A - 2 862 974**
**US - A - 3 271 441**
**US - A - 4 172 946**

**JOURNAL OF FLUORINE CHEMISTRY, Band 2, Juli 1972, Lausanne, P.L. COE und N.E. MILNER "(Perfluoralkyl)copper(I) compounds III. Reaction of (perfluoroalkyl)-copper(I) compounds with aromatic hydrocarbons", Seiten 167-172**
**JOURNAL OF FLUORINE CHEMISTRY, Band 17, Jänner 1981, Lausanne, A.B. COWELL und C. TAMBORSKI, "Fluoroalkylation of aromatic compounds", Seiten 345-356**
**PATENT ABSTRACTS OF JAPAN, unexamined applications, Field C, Band 6, Nr. 85, 22. Mai 1982, THE**

(73) Patentinhaber: **HOECHST AKTIENGESELLSCHAFT, Postfach 80 03 20, D-6230 Frankfurt am Main 80 (DE)**

(72) Erfinder: **von Werner, Konrad, Dr., Buch 1 1/2, D-8261 Halsbach (DE)**

(56) Entgegenhaltungen: (Fortsetzung)
**PATENT OFFICE JAPANESE GOVERNMENT, Seite 83 C 103**

EP 0 114 359 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft ein Verfahren zur Herstellung von perfluoralkylsubstituierten carbocyclischen oder heterocyclischen Verbindungen gemäß Anspruch 1.

Es ist bereits bekannt, carbocyclische Verbindungen, die gegebenenfalls Substituenten tragen können, wie Benzol, Toluol, Naphthalin, Halogenbenzol, mit Perfluoralkyliodiden bei 250°C ohne Anwesenheit weiterer Reaktionspartner umzusetzen. Neben der Schwierigkeit, bei vergleichsweise hohen Temperaturen und entsprechend hohen Drücken arbeiten zu müssen, ergibt diese thermische Perfluoralkylierung keine guten Ausbeuten, auch wenn mit einem Überschuß an carbocyclischer Verbindung gearbeitet wird. In neuerer Zeit wurde diese Reaktion in Gegenwart von Akzeptoren für Iodwasserstoff, beispielsweise Natriumacetat, durchgeführt, wobei die Ausbeute verbessert werden konnte, jedoch immer noch hohe Temperaturen (190 bis 250°C) notwendig waren. Kupfer(II), das dafür bekannt ist, freie Radikalarylierungen zu katalysieren, wie auch tertiäre Amine (beispielsweise Triethylamin), die die freie radikalische Addition von Perfluoralkyliodiden an Olefine katalysieren, sollen einen schädlichen Effekt auf die Reaktion haben (Journal of Fluorine Chemistry, 17 (1981), Seiten 345 bis 356, insbesondere Seite 347 oben).

Es ist ferner ein Verfahren bekannt, Perfluoralkyliodide mit carbocyclischen oder heterocyclischen Verbindungen in Gegenwart von mindestens äquimolaren Mengen einer freie Radikale erzeugenden Verbindung, beispielsweise organische Peroxide oder aliphatische Azoverbindungen umzusetzen. Weniger als äquimolare Mengen der freie Radikale erzeugenden Verbindungen können zwar verwendet werden, ergeben aber geringere Ausbeuten. Das Verfahren arbeitet bei niedrigeren Temperaturen (80 bis 190°C), hat aber den Nachteil, daß aus den freie Radikale erzeugenden Verbindungen Spaltprodukte und Umsetzungsprodukte sowohl mit Iod wie auch mit Wasserstoff entstehen, die die Aufarbeitung der Reaktionsmischung zum Erhalt der perfluoralkylierten, carbocyclischen oder heterocyclischen Verbindungen erschweren.

Weiterhin ist es bekannt, carbocyclische Verbindungen mit äquimolaren Mengen Perfluoralkyl-Kupfer in einem geeigneten Lösungsmittel, zum Beispiel Dimethylsulfoxid, bei vergleichsweise niedrigen Temperaturen (100 bis 120°C) umzusetzen. Eine katalytische Wirksamkeit des Kupfers soll hierbei ausgeschlossen sein. Eine Behandlung von Benzol mit Perfluoralkyliodid und Kupfer ohne vorhergehende Bildung von Perfluoralkyl-Kupfer soll nur geringe Ausbeuten an Perfluoralkylbenzol geben (Journal of Fluorine Chemistry 2 (1972/73), Seiten 167 bis 172, insbesondere Seite 169, Absatz 2). Nachteile dieses Verfahrens sind der erforderliche, relativ hohe Metallverbrauch, die Notwendigkeit, Lösungsmittel einsetzen zu müssen und die Tatsache, daß das gebildete Kupfer(I)-iodid in der Reaktionsmischung teilweise löslich ist, was die Abtrennung der perfluoralkylsubstituierten, carbocyclischen Verbindung wie auch die Aufarbeitung zur Rückgewinnung des verwendeten Lösungsmittels und des überschüssigen Kupfers erschwert.

Es wurde nun ein Verfahren gefunden, welches die Nachteile der bekannten Verfahren nicht aufweist und das es ermöglicht, bei mittleren Temperaturen, ohne die Notwendigkeit, Lösungsmittel verwenden zu müssen und ohne hohen Metallverbrauch perfluoralkylierte carbocyclische und heterocyclische Verbindungen in guten Ausbeuten herzustellen.

Dieses Verfahren zur Herstellung von perfluoralkylsubstituierten carbocyclischen oder heterocyclischen Verbindungen durch Reaktion von Perfluoralkyliodiden mit unsubstituierten oder substituierten, carbocyclischen oder heterocyclischen Verbindungen in der Wärme in Gegenwart mindestens eines basischen Salzes ist dadurch gekennzeichnet, daß die Reaktion in Gegenwart mindestens eines Metalls der ersten oder achten Nebengruppe des Periodensystems der Elemente oder in Gegenwart einer komplexen Verbindung, die ein solches Metall enthält, durchgeführt wird.

Als Perfluoralkyliodide sind Verbindungen der Formel

$$XR_FI$$

geeignet, in der bedeuten: X = F; Cl; Br; H und $R_F$ = ein geradkettiger Perfluoralkylenrest mit 1 bis 20 C-Atomen oder ein verzweigter Perfluoralkylenrest mit 3 bis 20 C-Atomen. Es können auch Mischungen von Perfluoralkyliodiden obengenannter Formel eingesetzt werden, die eine unterschiedliche Zahl von C-Atomen aufweisen. Wenn das Perfluoralkyliodid mehr als 20 C-Atome aufweist, wird im allgemeinen eine Verschlechterung der Ausbeute an perfluoralkylsubstituierten carbocyclischen bzw. heterocyclischen Verbindungen beobachtet. Vorzugsweise werden solche Perfluoralkyliodide obengenannter Formel verwendet, in denen X Fluor bedeutet.

Als unsubstituierte oder substituierte carbocyclische oder heterocyclische Verbindungen sind allgemein solche geeignet, die der $(4n + 2)$-$\pi$-Elektronenregel nach Hückel entsprechen. Beispiele für solche Verbindungen sind Benzol, Naphthalin, Anthracen, Phenanthren, Diphenyl, Pyridin, Chinolin, Furan und Thiophen. Sofern heterocyclische Verbindungen eingesetzt werden, die Stickstoffatome enthalten, sollten diese keine Bindungen mit Wasserstoffatomen enthalten, da in letzterem Fall eine vermehrte Bildung unerwünschter Nebenprodukte beobachtet wird.

Die für das erfindungsgemäße Verfahren einzusetzenden carbocyclischen oder heterocyclischen Verbindungen können einen oder mehrere Substituenten aufweisen, beispielsweise Alkylgruppen mit 1 bis

4 Kohlenstoffatomen, Alkoxygruppen mit 1 bis 4 Kohlenstoffatomen in der Alkylgruppe, Cyanogruppen, Fluor, Chlor, Brom sowie Oxy-, Carboxy- oder Sulfosäuregruppen. Sofern einer oder mehrere der drei letztgenannten Substituenten vorliegen, werden diese zweckmäßig als ihre Alkalisalze, beispielsweise als Natriumsalze, verwendet.

Sofern die Reaktion in Gegenwart mindestens eines Metalles der ersten oder achten Nebengruppe des Periodensystems der Elemente durchgeführt wird, wird die Reaktionsmischung vorteilhaft auf 110 bis 200°C erwärmt. Unterhalb 110°C ist die Reaktionsdauer sehr hoch. Es werden nicht die erwünschten guten Ausbeuten erzielt. Oberhalb 200°C wird im allgemeinen keine Ausbeuteverbesserung mehr beobachtet, die Zahl der gebildeten unerwünschten Nebenprodukte nimmt zu, der Energie- und Apparateaufwand steigt unnötig. Besonders gute Ergebnisse werden im Temperaturbereich von 130 bis 180°C erhalten.

Sofern die Reaktion in Gegenwart einer komplexen Verbindung, die mindestens ein Metall der ersten oder achten Nebengruppe des Periodensystems der Elemente enthält, durchgeführt wird, wird die Reaktionsmischung vorteilhaft auf 50 bis 140°C erwärmt. Unterhalb 50°C bzw. oberhalb 140°C treten ähnliche Schwierigkeiten auf, wie weiter oben beim Einsatz der entsprechenden Metalle unterhalb 110 bzw. oberhalb 200°C beschrieben. Bei Verwendung der komplexen Metallverbindungen werden besonders gute Ergebnisse erhalten, wenn im Temperaturbereich von 70 bis 120°C gearbeitet wird.

Die Reaktion soll in Gegenwart mindestens eines basischen Salzes ablaufen. Unter »basisches Salz« im Sinn der vorliegenden Erfindung ist ein Metallsalz gemeint, das in wäßriger Lösung oder Aufschlämmung basisch oder neutral reagiert und geeignet ist, in Mischung mit einer 0,1 normalen wäßrigen Salzsäure die Wasserstoffionen-Konzentration auf mindestens $^1/_{1000}$ des ursprünglichen Wertes herabzusetzen. Vorzugsweise werden als basische Salze Alkalimetall- oder Erdalkalimetall-Salze verwendet, vorteilhaft Salze des Natriums, Kaliums, Magnesiums oder Calciums. Beispiele für solche Salze sind die sekundären oder tertiären Phosphate von Natrium oder Kalium sowie die carbonsauren Salze, beispielsweise die Acetate von Natrium, Kalium, Magnesium oder Calcium. Besonders bevorzugt werden die Salze der Kohlensäure von Natrium, Kalium, Magnesium oder Calcium, oder das Oxid des Magnesiums oder des Calciums eingesetzt. Nicht geeignet sind Salze, deren Anion als sogenanntes »Katalysatorengift« für Metallkatalysatoren bekannt ist, beispielsweise die Sulfide oder Cyanide.

Von den basischen Salzen werden vorteilhaft 0,5 bis 2 Mol je Mol verwendeten Perfluoralkyliodid eingesetzt. Unter 0,5 Mol wird eine Verschlechterung der Ausbeute von den perfluoralkylsubstituierten, carbocyclischen oder heterocyclischen Verbindungen festgestellt. Oberhalb 2 Mol verwendetes basisches Salz tritt keine weitere Ausbeuteverbesserung ein, so daß höhere Zusätze im allgemeinen unnötig sind.

Die Reaktion wird in Gegenwart mindestens eines Metalles der ersten oder achten Nebengruppe des Periodensystems der Elemente oder in Gegenwart einer komplexen Verbindung, die ein solches Metall als Zentralatom enthält, durchgeführt. Unter »komplexe Verbindung« ist eine Koordinationsverbindung eines sogenannten Übergangsmetalles zu verstehen, wie es beispielsweise in »Advanced Inorganic Chemistry« (Cotton and Wilkinson) Interscience Publishers, 2. Auflage 1966, Seiten 124 und 125, definiert ist. Bevorzugt werden komplexe Verbindungen verwendet, in denen das Metall der ersten oder achten Nebengruppe des Periodensystems der Elemente als Zentralatom in der Oxidationsstufe 0, 1 oder 2 enthalten ist. Liegt das Metall in einer höheren Oxidationsstufe als 2 vor, wird ein Nachlassen der Wirksamkeit auf die Umsetzung von Perfluoralkyliodid mit carbocyclischen oder heterocyclischen Verbindungen beobachtet.

Gute Ergebnisse werden erhalten, wenn das Metall als Zentralatom in einer Komplexverbindung mit Triarylphosphin, Trialkoxyphosphin, Triaryloxyphosphin und/oder Kohlenoxid vorliegt, jedoch sind auch andere Liganden, beispielsweise Diene wie Cyclooctadien-(1,4), gut geeignet. Sofern das Metall als Zentralatom eine höhere Oxidationsstufe als 0 aufweist, sind beispielsweise die entsprechenden Metallhalogenide, insbesondere die Chloride, gut geeignet.

Wegen ihrer günstigen Wirksamkeit und leichten Zugänglichkeit werden vorzugsweise solche Metall-Komplexverbindungen eingesetzt, die Nickel oder Rhodium als Zentralatom enthalten. Beispiele für solche Verbindungen sind:

$(Ph_3P)_3RhCl$; $(Ph_3P)_2Rh(CO)Cl$; $[(CO)_2RhCl]_2$; $(COD)Rh(Cl_2)Rh(COD)$; $(Ph_3P)_2Ni(CO)_2$; $Ni(CO)_4$; $Ni[P(OC_2H_5)_3]_4$.

In den genannten Formeln bedeuten: Ph = Phenyl; COD = Cyclooctadien-(1,4). Die übrigen Buchstaben bzw. Buchstabenfolgen sind die üblichen Symbole für chemische Elemente.

Der Einsatz der oben näher beschriebenen Metallkomplexverbindungen für die Reaktion von Perfluoralkyliodiden mit carbocyclischen bzw. heterocyclischen Verbindungen ermöglicht die Anwendung vergleichsweise niedriger Reaktionstemperaturen. Es kann mit guten Ergebnissen im Temperaturbereich von 50 bis 140°C und insbesondere von 70 bis 120°C gearbeitet werden. Unter 50°C verläuft die Perfluoralkylierungsreaktion im allgemeinen zu langsam, es werden zu lange Reaktionszeiten beobachtet und ungünstige Ausbeuten. Oberhalb 140°C wird im allgemeinen keine Ausbeuteverbesserung mehr erzielt, es wächst die Gefahr von Nebenreaktionen, außerdem wäre ein unnötig hoher Energie-und Apparateaufwand notwendig. Die gewählte Reaktionstemperatur hängt unter anderem auch von der Größe der umzusetzenden Moleküle des Perfluoralkyliodids und der carbocyclischen bzw. heterocyclischen Verbindung andererseits ab, so daß im allgemeinen bei wachsender Molekülgröße höhere Reaktionstemperaturen vorteilhafter sind. Das gleiche gilt auch für die Perfluoralkylierungsreaktion in Gegenwart der reinen Metalle. Der hier zu wählende Temperaturbereich ist weiter oben beschrieben.

Da viele der vorteilhaft einzusetzenden komplexen Verbindungen, die ein Metall der ersten oder achten Nebengruppe des Periodensystems der Elemente als Zentralatom enthalten, oxidationsempfindlich sind, empfiehlt es sich, bei Einsatz solcher Verbindungen unter Inertgas, beispielsweise Stickstoff oder Argon, zu arbeiten. Der Zusatz eines Lösungsmittels ist im allgemeinen nicht erforderlich. Lediglich in den Fällen, wo ein Reaktionspartner bei der gewählten Reaktionstemperatur in der Reaktionsmischung im festen Zustand vorliegen würde, ist der Einsatz von polaren, aprotischen Lösungsmitteln wie Dimethylformamid, N-Methylpyrrolidon, Dimethylsulfoxid oder Tris-dimethylamino-phosphinoxid nützlich. Solche Lösungsmittel werden im allgemeinen eher bei niedrigeren Reaktionstemperaturen eingesetzt, wie sie bei der Durchführung der Reaktion mit Metallkomplexverbindungen angewendet werden.

Neben den weiter oben beschriebenen Metallkomplexverbindungen sind auch die reinen Metalle der ersten bzw. achten Nebengruppe des Periodensystems der Elemente für die Perfluoralkylierungsreaktion von carbocyclischen oder heterocyclischen Verbindungen wirksame Katalysatoren. Bei Anwendung der reinen Metalle müssen zwar höhere Reaktionstemperaturen angewendet werden, doch ergibt sich der Vorteil, daß die Metalle nach Beendigung der Reaktion leichter abgetrennt werden können. Vorteilhaft werden ein oder mehrere Metalle aus der ersten und achten Nebengruppe des Periodensystems der Elemente eingesetzt, die in der fünften oder sechsten Periode dieses Systems stehen. Wegen seiner guten Wirksamkeit und günstigen Beschaffbarkeit wird insbesondere Ruthenium als Metall verwendet.

Die genannten Metalle werden vorteilhaft in feiner Verteilung als Metallpulver oder noch besser auf einem inerten Träger, beispielsweise Aktivkohle oder Aluminiumoxid, niedergeschlagen verwendet.

Sowohl von den genannten Metallen wie auch von den Metallkomplexverbindungen brauchen nur katalytische Mengen eingesetzt zu werden. Gute Ergebnisse werden erzielt, wenn die Reaktion in Gegenwart von 0,001 bis 0,1 Mol des Metalles oder der Metallkomplexverbindung je Mol eingesetzten Perfluoralkyliodids durchgeführt wird. Unter 0,001 Mol ist im allgemeinen die Wirksamkeit zu gering, oberhalb 0,1 Mol wird keine Verbesserung der Wirksamkeit mehr festgestellt. Ein höherer Einsatz des Metalles bzw. der Metallkomplexverbindung wäre unnötig. Insbesondere wird die Reaktion in Gegenwart von 0,005 bis 0,05 Mol des Metalles oder der Metallkomplexverbindung durchgeführt.

Sofern die Siedepunkte (unter Normaldruck) der eingesetzten Reaktionspartner und der erzeugten Reaktionsprodukte oberhalb der gewählten Reaktionstemperatur liegen, kann in einem offenen System gearbeitet werden. Sofern ein Reaktionspartner bei der gewählten Reaktionstemperatur siedet, wird vorteilhaft am Rückfluß gekocht. Wenn die Siedepunkte niedriger liegen, wird im geschlossenen System unter dem autogenen Druck der Reaktionsmischung gearbeitet, die Anwendung zusätzlichen Druckes durch Zuführung von Inertgas ist nicht erforderlich.

Bei Durchführung des erfindungsgemäßen Verfahrens soll das Molverhältnis des Perfluoralkyliodids zur carbocyclischen oder heterocyclischen Verbindung mindestens 1 : 1 betragen, zweckmäßig wird ein Überschuß der carbocyclischen bzw. heterocyclischen Verbindung eingesetzt, wodurch im allgemeinen eine Ausbeuteverbesserung an perfluoralkyliertem Produkt erzielt wird. Ein Molverhältnis von Perfluoralkyliodid zur carbocyclischen bzw. heterocyclischen Verbindung von über 1 : 10 bringt im allgemeinen keine weitere Ausbeuteverbesserung und ist nur dann zweckmäßig, wenn die carbocyclische oder heterocyclische Verbindung billig ist und in der großen Menge als Lösungsmittel notwendig. Vorzugsweise wird bei einem Molverhältnis von Perfluoralkyliodid zur carbocyclischen bzw. heterocyclischen Verbindung von 1 : 2 bis 1 : 5 gearbeitet. Ein molarer Überschuß der Perfluoralkyliodids gegenüber der carbocyclischen bzw. heterocyclischen Verbindung würde eine Disubstitution begünstigen, die im allgemeinen nicht erwünscht ist, außerdem können insbesondere beim Einsatz von carbocyclischen oder heterocyclischen Verbindungen mit Seitenketten an diesen Perfluoralkylierungen auftreten.

Durch das erfindungsgemäße Verfahren wird eine bevorzugte einfache Perfluoralkylierung am Kern der carbocyclischen bzw. heterocyclischen Verbindung erreicht. Die Ausbeute an solchen einfach substituierten Verbindungen ist gut, die anzuwendenden Reaktionstemperaturen liegen vergleichsweise im mittleren bis unteren Bereich. Insbesondere bei Einsatz der reinen Metalle als Katalysatoren erfolgt die Trennung vom Reaktionsgemisch und der Wiedereinsatz der Katalysatoren einfach und ohne großen Aufwand. Während der Umsetzung des Perfluoralkyliodids mit der carbocyclischen bzw. heterocyclischen Verbindung sollte die Reaktionsmischung bewegt werden, beispielsweise durch Rühren oder Schütteln. Dies gilt besonders bei Anwendung der Metalle als Katalysatoren.

Der Ausdruck »reine Metalle« in der vorangegangenen Beschreibung wird zum Unterschied von den Metallkomplexverbindungen gebraucht. Er bedeutet nicht, daß nicht auch Metall-Legierungen für das neue Verfahren geeignet sind.

Zur Durchführung des erfindungsgemäßen Verfahrens werden die oben beschriebenen Reaktionskomponenten in den angegebenen Mengen, je nach den eingesetzten Verbindungen und angewendeten Reaktionstemperaturen, bei Normaldruck, unter Verwendung eines Rückflußkühlers, gerührt, oder unter Rühren am Rückfluß gekocht, oder in einem Autoklav gerührt bzw. geschüttelt. Die Reaktionsdauer liegt im allgemeinen zwischen einer und vierzig Stunden. Nach Beendigung der Reaktion wird abgekühlt, gegebenenfalls entspannt, das Reaktionsgemisch filtriert, der Filterkuchen mit der gleichen carbocyclischen oder heterocyclischen Verbindung, wie sie für die Reaktion verwendet wurde, gewaschen, sofern diese Verbindung flüssig ist, wenn nicht, wird mit dem für die Reaktion verwendeten Lösungsmittel gewaschen, die erhaltene Waschflüssigkeit mit dem Filtrat vereinigt und destillativ aufgearbeitet. Hierbei verbleiben Katalysatorreste im Destillationsrückstand.

Aus dem Filterrückstand wird das gebildete, in der Reaktionsmischung unlösliche Iodid durch Extrahieren mit Wasser oder einem anderen geeigneten Lösungsmittel, abgetrennt und das Iod nach üblichen Methoden wiedergewonnen. Sofern ein im Reaktionsgemisch unlöslicher Katalysator verwendet wurde, wird dieser nach Abtrennung des gebildeten Iodids, falls erforderlich, gereinigt und wieder eingesetzt.

Das erfindungsgemäße Verfahren besitzt gegenüber den rein thermischen Verfahren den Vorteil, bei niedrigeren Temperaturen und mit höherer Selektivität abzulaufen.

Dadurch ergeben sich bei empfindlichen carbocyclischen bzw. heterocyclischen Verbindungen weniger Nebenprodukte, während bei stabilen carbocyclischen bzw. heterocyclischen Verbindungen das für die Erzielung gleich guter Ausbeuten erforderliche Molverhältnis von carbocyclischer bzw. heterocyclischer Verbindung zum Perfluoralkyliodid reduziert werden kann. Bei Einsatz von Metallkatalysatoren, die im Reaktionsgemisch unlöslich sind, gestalten sich die Wiedergewinnung und der Wiedereinsatz besonders einfach.

Mit dem erfindungsgemäßen Verfahren können Produkte erzeugt werden, die wertvolle Zwischenprodukte für die Herstellung von Tensiden, Pharmaka, Farbstoffen und Pflanzenschutzmitteln sind.

Nachfolgende Beispiele sollen die Erfindung näher erläutern. Die Molverteilung der in den Rohprodukten vorliegenden Stoffe wurde an filtrierten Proben durch $^{19}F$-Kernresonanz ermittelt, die Isomerenverteilung an destillativ gereinigten Produkten durch kombinierte Auswertung von gaschromatographisch erhaltenen Werten sowie der $^{19}F$-,$^{1}H$-Kernresonanzspektren.

Beispiel 1

Herstellung von Perfluoroctyl-benzol

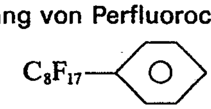

In einen Autoklav von 4 $dm^3$ Inhalt aus rostfreiem Stahl werden eingefüllt:

1638 g (3,0 Mol) $C_8F_{17}I$, 1172 g (15,0 Mol) Benzol,
228 g (1,65 Mol $K_2CO_3$) und 124 g Ruthenium auf Aktivkohle, entsprechend 0,06 Mol Ru (2 Mol-%, bezogen auf $R_FI$).

Die Mischung wird 30 Stunden bei 170°C geschüttelt, wobei sich ein Enddruck von 28 bar einstellt. Nach Abkühlen auf Raumtemperatur wird entspannt und das Gemich filtriert. Der Autoklav wird mit 200 ml Benzol ausgespült und dieses zum Nachwaschen des Filterkuchens verwendet. Das $^{19}F$-Kernresonanzspektrum der vereinigten Filtrate ergibt eine Produktverteilung von 95,2 Mol-% $C_8F_{17}$—$C_6H_5$ und 4,8 Mol-% $C_8F_{17}H$, also einen vollständigen Umsatz des $C_8F_{17}I$. Nach Abdestillieren des Benzols und einer $C_8F_{17}H$-haltigen Zwischenfraktion werden 1328 g $C_8F_{17}$—$C_6H_5$ mit Siedepunkt 92 bis 93°C bei 1,33 kPa erhalten (Ausbeute 89,2%, Reinheit nach Gaschromatographie: 99,7%).

Zur Wiedergewinnung des Katalysators wird der Filterrückstand mit 1 l siedendem Wasser gerührt, filtriert, dann nochmals unter Einleiten von Dampf ausgekocht, abfiltriert und im Vakuum bei 50°C getrocknet. Durch Ansäuern der wäßrigen Filtrate mit Schwefelsäure und Versetzen mit 30%iger $H_2O_2$-Lösung wird elementares Iod ausgefällt, welches abfiltriert und gewaschen wird. Die Gesamtmenge an Iod, angegeben als Trockengehalt aufgrund des durch Titration einer Probe ermittelten $I_2$-Gehalts, beträgt 371 g, was einer fast quantitativen Rückgewinnung entspricht.

Beispiel 2

Perfluoroctyl-benzoesäuremethylester

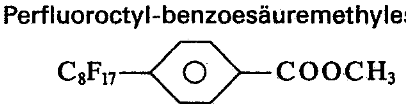

Ein Gemisch aus 818 g $C_8F_{17}I$ (1,5 Mol), 1021 g Benzoesäuremethylester (7,5 Mol), 110,5 g $K_2CO_3$ (0,8 Mol) und 61,9 g Ruthenium auf Aktivkohle, entsprechend 0,03 Mol Ru (2 Mol-%, bezogen auf $R_FI$) wird in einem Autoklav von 4 $dm^3$ Inhalt während 20 Stunden bei 160°C kräftig gerührt. Nach Abkühlen und Entspannen wird filtriert, das $^{19}F$-Kernresonanzspektrum des Filtrats ergibt Molanteile von 75% $C_8F_{17}$—$C_6H_4$—$COOCH_3$ und 25% $C_8F_{17}H$. Der abfiltrierte Feststoff wird mit 500 ml $CH_3OH$ ausgekocht.

Die vereinigten Filtrate werden anfangs bei Normaldruck, dann im Vakuum destilliert. Der Hauptlauf mit Siedepunkt 107 bis 109°C bei 1,33 Pa wird im Ölpumpenvakuum abdestilliert. Man erhält 525,4 g farbloses Produkt, welches sofort in der gekühlten Vorlage erstarrt (Ausbeute 63,2%).

## Beispiele 3 bis 7

Es wird verfahren wie im Beispiel 1 beschrieben, wobei die in nachfolgender Tabelle I angeführten Metall-Katalysatoren und Perfluoralkyliodide in den ebenfalls verzeichneten Mengenverhältnissen eingesetzt werden. Die erzielte Zusammensetzung des Rohproduktes sowie die Ausbeuten an $R_F$—$C_6H_5$ sind aus der Tabelle ersichtlich. Die in der zweiten senkrechten Spalte angegebenen Metalle werden in feiner Verteilung auf Aktivkohle eingesetzt mit Ausnahme des Goldes, welches als Metallpulver angewendet wird.

Die hinter den Metall-Symbolen stehenden Zahlen geben an, wieviel Mol des Metalles, bezogen auf 100 Mol eingesetztes $R_FI$, verwendet werden.

Tabelle I

| Beispiel Nr. | Metall (Mol-% $R_FI$) | $R_FI$ | (Mol) | $C_6H_6$ (Mol) | $K_2CO_3$ (Mol) | Zeit (h) | Temp. (°C) | Rohprodukt (Mol-%) $R_FC_6H_5$ | $R_FI$ | $R_FH$ | Isoliertes $R_F$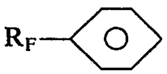 a) | (%) b) |
|---|---|---|---|---|---|---|---|---|---|---|---|---|
| 3 | Au 1 | $C_6F_{13}I$ | 0,2 | 1,0 | 0,11 | 20 | 160 | 73 | 25 | 2 | 63 | 84 |
| 4 | Ru 2 | $C_6F_{13}I$ | 1,0 | 5,0 | 0,55 | 30 | 160 | 84 | 15 | 1 | 78 | 92 |
| 5 | Pt 1 | $C_{10}F_{21}I$ | 0,1 | 0,5 | 0,06 | 20 | 170 | 76 | 9 | 15 | 62 | 68 |
| 6 | Pt 1 | $R_FI$ c) | 0,2 | 1,0 | 0,11 | 20 | 170 | 55 | 38 | 7 | 50 | 81 |
| 7 | Pt 2 | $(CF_3)_2CFI$ | 0,2 | 1,0 | 0,11 | 30 | 170 | 75 | 23 | 2 | 61 | 81 |

a) Ausbeute, bezogen auf eingesetztes Perfluoralkyliodid,
b) bezogen auf umgesetztes $R_FI$,
c) $R_FI$: 40% $C_6$, 35% $C_8$, 17% $C_{10}$, 8% $C_{12}$

Siedepunkte von $R_F$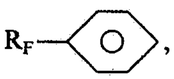, worin bedeuten

| | |
|---|---|
| $R_F = C_2F_5$ | 108°C/100 kPa |
| $C_6F_{13}$ | 65 bis 67°C/1,3 kPa |
| $C_8F_{17}$ | 92 bis 93°C/1,3 kPa |
| $C_{10}F_{21}$ | 112 bis 115°C/1,3 kPa |
| $(CF_3)_2CF$ | 119 bis 121°C/101 kPa |

Beispiele 8 bis 14

Es wird ähnlich verfahren wie im Beispiel 2 beschrieben, wobei stets ein Überschuß von 10 Mol-%, bezogen auf eingesetztes Perfluoralkyliodid, von Kaliumcarbonat verwendet wird. Wie im Beispiel 2 wird Ruthenium auf Aktivkohle als Katalysator in einer Menge von 2 Mol-%, bezogen auf eingesetztes Perfluoralkyliodid, verwendet. Art und Menge der Reaktionspartner, Temperatur, Reaktionszeit sowie Analyse des Reaktions-Rohproduktes, Ausbeute, bezogen auf eingesetztes Perfluoralkyliodid und Siedepunkt der erhaltenen perfluoralkylierten, carbocyclischen bzw. heterocyclischen Verbindung, sind in nachfolgender Tabelle II angegeben.

In dieser Tabelle wird die Kurzbezeichnung »Aromat« bzw. in der Formel das Symbol »Ar« für die jeweils angegebene carbocyclische oder heterocyclische Verbindung verwendet.

Tabelle II

| Beispiel Nr. | $R_fI$ | (Mol) | Aromat | (Mol) | Zeit (h) | Temp. (°C) | Rohprodukt (Mol-%) $R_F$–Ar | $R_FI$ | $R_FH$ | Ausbeute[a]) (%) | Siedepunkt (°C/kPa) | Isomerenverteilung (%) 2 | 3 | 4 |
|---|---|---|---|---|---|---|---|---|---|---|---|---|---|---|
| 8 | $C_4F_9I$ | 0,2 | 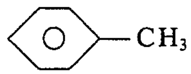 | 1,0 | 25 | 160 | —— nicht ermittelt —— | | | 47 | 85/6,6 | 25 | 44 | 31 |
| 9 | $C_8F_{17}I$ | 0,2 | 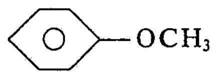 | 1,0 | 30 | 170 | 67 | – | 33 | 55 | 77-80/0,026 | 19 | 50 | 31 |
| 10 | $C_6F_{13}I$ | 0,1 | 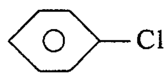 | 0,5 | 20 | 170 | 64 | 23 | 13 | 57 | 86-90/2 | 11 | 89[b]) | – |
| 11 | $C_8F_1$-I | 1,5 | 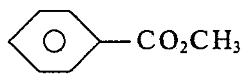 | 7,5 | 20 | 160 | 75 | – | 25 | 63 | 107-110/0,0013 | 5 | 15 | 80 |
| 12 | $C_6F_{13}I$ | 0,2 | 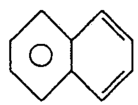 | 0,6 | 20 | 150 | 100 | – | – | 69 | 135-140/1,7 | 79[c]) | 21[c]) | – |
| 13 | $C_8F_1$-I | 0,2 | 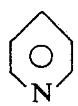 | 1,0 | 20 | 150 | 95 | – | 5 | 51 | 99-105/1,3 | 53 | 36 | 11 |
| 14 | $C_6F_{13}I$ | 0,1 | 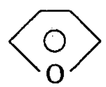 | 0,5 | 20 | 150 | 64 | 36 | – | 46 | 76/6,6 | 100 | – | – |

[a]) Bezogen auf eingesetztes $R_FI$.
[b]) m- und p-Isomere konnten durch Gaschromatographie nicht getrennt werden. In der Hochdruck-Flüssigkeitschromatographie zeigt Hauptpeak eine Schulter.
[c]) Nomenklaturgerecht sind hier 1- bzw. 2-Isomeres gemeint.

Beispiele 15 bis 19

0,1 Mol $C_6F_{13}I$, 0,2 Mol Benzol, 0,055 Mol Kaliumcarbonat und 2 Mol-%, bezogen auf eingesetztes $C_6F_{13}I$, des in nachfolgender Tabelle angegebenen Katalysators, dem bei den Beispielen 16, 17 und 19 noch 1 bzw. 2 Mol-%, bezogen auf eingesetztes $C_6F_{13}I$, an Zinkpulver als Reduktionsmittel zugegeben werden, werden in einem Glaskolben 7 Stunden unter Rühren in Argon als Schutzgasatmosphäre bei Normaldruck gekocht. Nach dem Abkühlen wird filtriert, der Filterrückstand zweimal mit Benzol gewaschen, das Filtrat und die Waschflüssigkeit mit wäßriger 10gewichtsprozentiger Salzsäure zweimal, dann mit wäßriger 5gewichtsprozentiger Kaliumcarbonatlösung einmal ausgeschüttelt und nach Trocknen über entwässertem Natriumsulfat das Reaktions-Rohprodukt, wie oben beschrieben, analysiert. Anschließend wrd durch fraktionierte Destillation das gebildete $C_6F_{13}$—$C_6H_5$ abgetrennt und dessen Ausbeute, bezogen auf den $R_F$-Umsatz, bestimmt. Die erhaltenen Werte sind in nachfolgender Tabelle III angegeben.

Tabelle III

| Beispiel Nr. | Katalysator | Rohprodukt (Mol-%) $R_FI$ | $R_F$—$C_6H_5$ | $R_FH$ | $C_6F_{13}$—$C_6H_5$, bez. auf $R_FI$-Umsatz Ausbeute (%) (nach Destillation) |
|---|---|---|---|---|---|
| 15 | $(Ph_3P)_2Ni(CO)_2$ | 67 | 33 | – | 75 |
| 16 | $(Ph_3P)_2Ni(CO)_2$ + 1 Mol-% Zn | 60 | 40 | – | 79 |
| 17 | $(Ph_3P)_2NiBr_2$ + 2 Mol-% Zn | 59,5 | 40,5 | – | 73 |
| 18 | $(Ph_3P)_3RhCl$ | 82 | 18 | – | 51 |
| 19 | $(Ph_3P)_3RhCl$ + 1 Mol-% Zn | 68 | 32 | Spuren | 68 |

Beispiel 20

Das Beispiel 19 wird in einem Autoklav bei 120°C und 7 Stunden Reaktionsdauer wiederholt. Hierbei wird ein Reaktions-Rohprodukt folgender Zusammensetzung erhalten:

36,5 Mol-% $R_FI$; 52,5 Mol-% $R_F$—$C_6H_5$;
3 Mol-% $R_FH$ und 8% einer unbekannten Substanz.

**Patentansprüche**

1. Verfahren zur Herstellung von perfluoralkylsubstituierten carbocyclischen oder heterocyclischen Verbindungen durch Reaktion von Perfluoralkyliodiden mit unsubstituierten oder substituierten carbocyclischen oder heterocyclischen Verbindungen in der Wärme, in Gegenwart mindestens eines basischen Salzes, dadurch gekennzeichnet, daß die Reaktion in Gegenwart mindestens eines Metalles der 1. oder 8. Nebengruppe des Periodensystems der Elemente oder in Gegenwart einer komplexen Verbindung, die ein solches Metall als Zentralatom enthält, durchgeführt wird.

2. Verfahren nach Anspruch 1, dadurch gekennzeichnet, daß die Reaktion in Gegenwart von 0,5 bis 2 Mol eines basischen Salzes je Mol verwendetes Perfluoralkyliodid durchgeführt wird.

3. Verfahren nach Anspruch 1 oder 2, dadurch gekennzeichnet, daß als basisches Salz ein Salz der Kohlensäure von Natrium, Kalium, Magnesium oder Calcium oder das Oxid des Magnesiums oder Calciums verwendet wird.

4. Verfahren nach einem oder mehreren der Ansprüche von 1 bis 3, dadurch gekennzeichnet, daß die Reaktion in Gegenwart von 0,001 bis 0,1 Mol des Metalles oder der Metallkomplexverbindung je Mol eingesetztes Perfluoralkyliodid durchgeführt wird.

5. Verfahren nach einem oder mehreren der Ansprüche von 1 bis 4, dadurch gekennzeichnet, daß die Reaktion in Gegenwart eines Metalles aus der 5. oder 6. Periode des Periodensystems der Elemente durchgeführt wird.

6. Verfahren nach einem oder mehreren der Ansprüche von 1 bis 5, dadurch gekennzeichnet, daß das Metall in feinverteilter Form auf einem inerten Träger niedergeschlagen ist.

7. Verfahren nach einem oder mehreren der Ansprüche von 1 bis 6, dadurch gekennzeichnet, daß als Metall Ruthenium verwendet wird.

8. Verfahren nach einem oder mehreren der Ansprüche von 1 bis 7, dadurch gekennzeichnet, daß die Reaktion bei 110 bis 200°C durchgeführt wird.
9. Verfahren nach einem oder mehreren der Ansprüche von 1 bis 4, dadurch gekennzeichnet, daß das Metall in der Oxidationsstufe 0 bis 2 als Zentralatom einer Komplexverbindung vorliegt.
10. Verfahren nach einem oder mehreren der Ansprüche von 1 bis 4 und 9, dadurch gekennzeichnet, daß das Metall als Komplexverbindung mit Triarylphosphin, Triaryloxyphosphin, Trialkoxyphosphin und/oder Kohlenoxid vorliegt.
11. Verfahren nach Anspruch 9 oder 10, dadurch gekennzeichnet, daß die Reaktion unter Inertgas bei 50 bis 140°C durchgeführt wird.

## Claims

1. A process for the manufacture of perfluoralkylsubstituted carbocyclic or heterocyclic compounds by reaction of perfluoralkyl-iodides with unsubstituted or substituted carbocyclic or heterocyclic compounds at elevated temperatures and in presence of at least one alkaline salt, characterised by carrying out the reaction in the presence of at least one metal of the first or eight auxiliary group of the periodic table or in the presence of a complex compound containing said metal as the central atom.
2. The process as claimed in claim 1, characterised by carrying out the reaction in presence of from 0.5 to 2 moles of an alkaline salt, relative to one mole of the perfluoroalkyl-iodide being employed in the reaction.
3. The process as claimed in either of claims 1 or 2, characterised in that a salt of the carbonic acid of sodium, potassium, magnesium or calcium or the oxide of magnesium or calcium is employed as the alkaline salt.
4. The process as claimed in one or more of claims 1 to 3, characterised by carrying out the reaction in presence of from 0.001 to 0.1 mole of the metal or of the metal complex compound, relative to one mole of the perfluoroalkyl iodide being employed in the reaction.
5. The process as claimed in one or more of claims 1 to 4, characterised by carrying out the reaction in presence of a metal from the fifth or sixth period of the periodic table.
6. The process as claimed in one or more of claims 1 to 5, characterised in that the metal has been precipitated onto an inert carrier material in finely divided particles.
7. The process as claimed in one or more of claims 1 to 6, characterised in that ruthenium is employed as the metal.
8. The process as claimed in one or more of claims 1 to 7, characterised by carrying out the reaction at a temperature of from 110 to 200°C.
9. The process as claimed in one of more of claims 1 to 4, characterised in that the metal being the central atom in the complex compound has an oxidation number of from 0 to 2.
10. The process as claimed in one or more of claims 1 to 4 and 9, characterised in that the metal is in a complex compound with triaryl-phosphine, triaryloxy-phosphine, trialkyl-phosphine, trialkoxy-phosphine, and/or carbon monoxide.
11. The process as claimed in either of claims 9 or 10, characterised by carrying out the reaction in an inert gas atmosphere at a temperature of from 50 to 140°C.

## Revendications

1. Procédé de préparation de composés carbocycliques ou hétérocycliques perfluoralkylés, par réaction d'iodures de perfluoralkyles avec des composés carbocycliques ou hétérocycliques substitués ou non, à chaud et en présence d'au moins un sel basique, procédé caractérisé en ce qu'on effectue ladite réaction en présence d'au moins un métal du premier ou du huitième sous-groupe de la classification périodique des éléments, ou en présence d'un complexe contenant un tel métal comme atome central.
2. Procédé selon la revendication 1 caractérisé en ce qu'on effectue la réaction en présence de 0,5 à 2 moles d'un sel basique par mole de l'iodure de perfluoralkyle utilisé.
3. Procédé selon l'une des revendications 1 et 2, caractérisé en ce qu'on utilise, comme sel basique, un sel formé par l'acide carbonique avec le sodium, le potassium, le magnésium ou le calcium, ou l'oxyde de magnesium ou de calcium.
4. Procédé selon l'une quelconque des revendications 1 à 3, caractérisé en ce qu'on effectue la réaction en présence de 0,001 à 0,1 mole du métal ou du complexe métallique par mole de l'iodure de perfluoralkyle mis en jeu.
5. Procédé selon l'une quelconque des revendications 1 à 4, caractérisé en ce qu'on effectue la réaction en présence d'un métal de la cinquième période ou de la sixième période de la classification périodique des éléments.
6. Procédé selon l'une quelconque des revendications 1 à 5, caractérisé en ce que le métal est déposé à l'état finement divisé sur un support inerte.

7. Procédé selon l'une quelconque des revendications 1 à 6, caractérisé en ce qu'on utilise le ruthénium comme métal.

8. Procédé selon l'une quelconque des revendications 1 à 7, caractérisé en ce qu'on effectue la réaction à une température de 110 à 200°C.

9. Procédé selon l'une quelconque des revendications 1 à 4, caractérisé en ce que le métal se trouve à un degré d'oxydation de 0 à 2 en tant qu'atome central d'un complexe.

10. Procédé selon l'une quelconque des revendications 1 à 4 et 9, caractérisé en ce que le métal se trouve sous la forme d'un complexe formé avec une triarylphosphine, une triaryloxyphosphine, une trialcoxyphosphine et/ou l'oxyde de carbone.

11. Procédé selon l'une des revendications 9 et 10, caractérisé en ce que la réaction est effectuée sous un gaz inerte, à une température de 50 à 140°C.